# EUROPEAN PATENT APPLICATION

(11) **EP 3 730 601 A1**
(43) Date of publication of application: **28.10.2020**
(21) Application number: 18904203.9
(22) Date of filing: 04.12.2018
(51) Int. Cl.: C12M 1/26, G02B 21/32

(54) **MOVEMENT METHOD AND MOVEMENT DEVICE FOR BIOLOGICAL SUBJECT**

(30) Priority: 31.01.2018 JP 2018015010
(71) Applicant: Yamaha Hatsudoki Kabushiki Kaisha, Iwata-shi, Shizuoka-ken 438-8501 (JP)
(72) Inventor: SAKAMOTO, Masaru, Iwata-shi, Shizuoka-ken 438-8501 (JP)
(74) Representative: Müller-Boré & Partner Patentanwälte PartG mbB
(86) International application number: PCT/JP2018/044630
(87) International publication number: WO 2019/150756

(57) **Abstract**

A cell transfer device (S) transfers cells (C) from a transfer source dish (2) to wells (41) of a microplate (4). The cell transfer device (S) includes a camera unit (5) for acquiring condition information such as an area of the cells by imaging the dish (2) holding the cells (C); a head (61) to which a tip (12) that picks up the cells is attached and which transfers the picked up cells to the wells (41); and a control unit (7) which controls operation of the head (61). The control unit (7) refers to the cell condition information acquired by the camera unit (5) and causes the head (61) to transfer cells to at least two wells among the plurality of wells (41) provided in the microplate (4) so that the cells are housed in a state under the same condition.

## Description

### Technical Field

The present invention relates to a method and a device for transferring a biological subject such as a cell or a cell clusters to a container having a plurality of wells from a predetermined holding position.

### Background Art

For example, in applications for medical care or biological research, there may be conducted work of selecting a biological subject such as a cell or a cell clusters in a transfer source container and transferring the selected biological subject to a microplate including a plurality of wells. For example, there may be conducted work of capturing, by an image device, an image of cells scattered on a selection plate having numerous housing recesses, selecting a desired cell based on the obtained image, and transferring the selected cell to each well of the microplate after suctioning the cell by a tip (e.g. Patent Literature 1). In the microplate, such processing as a medical effect test or the like is conducted in which a medicine to be tested is added to the cells transferred to the plurality of wells and the cells are cultured.

Work of transferring cells to the microplate has been conventionally given consideration that the number of cells to be input to each well is roughly made uniform by using a statistical method. This is intended for uniformizing a state of a cell housed in each well in order to evaluate efficacy of a medicine in each well under as similar a condition as possible. However, in a case, for example, where efficacy of a medicine test or the like is conducted for three-dimensionally cultured cells, uniformizing each well is hard to be realized just by roughly making the number of cells uniform. This is because three-dimensionally cultured cells are likely to have non-uniform shapes, so that distributing cells to each well simply dependently on the number of cells causes a state of a cell to vary with each well.

### Citation List

### Patent Literature

Patent Literature 1: WO No. 2015/087371 A

### Summary of Invention

An object of the present invention is to provide a transfer method and a transfer device for a biological subject, which enable evaluation of a biological subject in each well of a plurality of wells under the same condition when biological subjects are transferred to the plurality of wells to conduct processing such as a medical effect test.

A transfer method for a biological subject according to one aspect of the present invention is a biological subject transfer method including picking up a biological subject at a predetermined holding position; and transferring the picked up biological subject to a container having a plurality of wells, in which biological subjects are transferred to at least two wells among the plurality of wells so that the biological subjects are housed in a state under the same condition.

A transfer device for a biological subject according to another aspect of the present invention includes an information acquisition unit which acquires condition information of a biological subject at a predetermined holding position; a head having a tip which picks up the biological subject and capable of transferring the picked up biological subject to a container having a plurality of wells; and a control unit which controls operation of the head, in which the control unit refers to condition information of the biological subject acquired by the information acquisition unit and causes the head to transfer biological subjects to at least two wells among the plurality of wells so that the biological subjects are housed in a state under the same condition.

### Brief Description of Drawings

FIG. 1 is a view schematically showing an example of a configuration of a cell transfer device to which a transfer method and a transfer device for a biological subject according to the present invention are applied.
FIG. 2A is a perspective view of a microplate for use in the cell transfer device and FIG. 2(B) is a vertical sectional view of FIG. 2A.
FIG. 3A is a schematic view showing a state of cells housed in wells in a comparative example and FIG. 3B and FIG. 3C are schematic views showing a state of cells housed in wells in the present embodiment.
FIG. 4 is a schematic view for describing a concept of bringing an amount of cells housed in each well into the same state in the present embodiment.
FIG. 5 is a view showing an example of an image for calculating an area of a cell.
FIGs. 6A and 6B are views for describing a method of capturing an image of a cell for obtaining a volume of a cell.
FIG. 7 is a view showing an example of uniformizing cells housed in each well based on intensities of fluorescence of the cells.
FIG. 8 is a block diagram showing a configuration of the cell transfer device.
FIG. 9 is a flow chart showing cell transfer operation using the cell transfer device.
FIG. 10 is a top view of the microplate, and is a view for describing an example of wells in which cells are housed in a state under the same condition.

### Description of Embodiment

In the following, an embodiment of the present invention will be described in detail with reference to the drawings. In a transfer method and a transfer device for a biological subject according to the present invention, various biological subjects can be used as a transfer target. As a biological subject to which the present invention is applicable, a typical example is an organism-derived cell. Examples of the organism-derived cell here include a single cell (cell) such as a blood cell or a singled cell, tissue fragment such as Histoculture and CTOS, cell aggregation cluster such as spheroid and organoid, individuals such as zebrafish, nematode, and fertilized egg, and a two-dimensional or three-dimensional colony. In addition to these examples, tissues, microorganisms, small species, and the like can be exemplified as biological subjects. In the embodiment to be described below, an example is shown in which a biological subject is a cell or a cell aggregation cluster formed by aggregating several to several hundred thousands of cells (hereinafter, collectively referred to simply as a "cell C").

### [Overall Configuration of Cell Transfer Device]

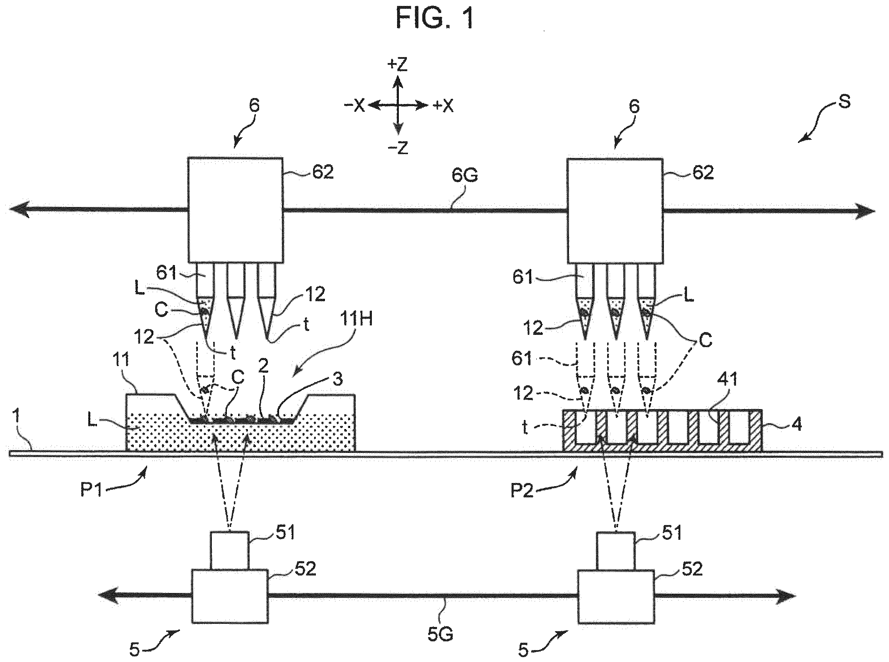

FIG. 1 is a view schematically showing an overall configuration of a cell transfer device S (a transfer device for a biological subject) to which a transfer method for a biological subject according to the present invention are applied. Here, the cell transfer device S which transfers a cell C between two containers (a dish 2 and a microplate 4) is exemplified.

The cell transfer device S includes a light transmissive base 1 having a horizontal mounting surface (an upper surface), a camera unit 5 (an imaging unit) arranged below the base 1, and a head unit 6 arranged above the base 1. At a first mounting position PI of the base 1, a selection container 11 provided with a dish 2 (at a predetermined holding position) is mounted and at a second mounting position P2, a microplate 4 (a container having a plurality of wells) is mounted. The head unit 6 includes a plurality of heads 61 movable along a Z direction (an up-down direction) Tips 12 for suctioning and discharging the cell C are attached to the heads, respectively. The camera unit 5 and the head unit 6 are movable in an X direction (a horizontal direction) and a direction (a Y direction) vertical to the plane of FIG. 1. The dish 2 and the microplate 4 are mounted on the upper surface of the base 1 within a movable range of the head unit 6.

Roughly described, the cell transfer device S is a device which individually picks up the cells C by each of the plurality of tips 12 from the dish 2 of the selection container 11 which holds numerous cells C, and transfers the picked up cells C to the microplate 4, and also simultaneously discharges the cells C from the plurality of tips 12 to the microplate 4 (wells 41). Before suction of the cell C, an image of the cell C held in the dish 2 is captured by the camera unit 5 to conduct selection work of selecting a good quality cell C to be transferred to the microplate 4.

Each part of the cell transfer device S will be described in the following. The base 1 is a rectangular flat plate having a predetermined rigidity and formed, in part or in entirety, of a light transmissive material. The base 1 is preferably a glass plate. Forming the base 1 of such a light transmissive material as a glass plate allows the camera unit 5 arranged below the base 1 to capture an image of the selection container 11 (the dish 2) and the microplate 4 arranged on the upper surface of the base 1 through the base 1.

The selection container 11 is a container as a transfer source of the cell C, and retains a medium L and holds the cell selection dish 2 being immersed in the medium L. The dish 2 is a plate which holds the cells C and has, on its upper surface, holding recesses 3 capable of individually housing and holding the cells C. The medium L is not particularly limited and any medium that will not deteriorate properties of the cell C can be appropriately selected according to a kind of the cell C.

The selection container 11 is provided, on its upper surface side, with a rectangular upper opening 11H. The upper opening 11H is an opening for injecting the cell C and picking up the selected cell C. The dish 2 is arranged below the upper opening 11H. The selection container 11 and the dish 2 for use are made of a light translucent resin material or glass. This is for enabling the camera unit 5 arranged below the selection container 11 to observe the cell C held in the dish 2.

A plurality of the cells C being dispersed in a cell culture solution are injected to the selection container 11 from dispensation tips (not shown). The dispensation tip suctions, from the container which retains the cell culture solution containing a large amount of the cells C, the cell culture solution together with the cells C, and holds the cell culture solution and the cells in the dispensation tip. Thereafter, the dispensation tip is moved to a position over the selection container 11 to access the upper surface of the dish 2 through the upper opening 11H. Then, with a distal end opening of the dispensation tip immersed in the medium L in the selection container 11, the cell C held in the dispensation tip is discharged onto the dish 2 together with the cell culture solution.

The microplate 4 is a container which becomes a transfer destination of the cells C, and has a plurality of the wells 41 to which the cells C are discharged. The well 41 is a bottomed hole which is opened in an upper surface of the microplate 4. In one well 41, a necessary number (generally one) of the cells C are housed together with the medium L. The microplate 4 used here is also made of a light transmissive resin material or glass. This is for enabling the camera unit 5 arranged below the microplate 4 to observe the cell C held in the well 41.

The camera unit 5, which captures an image of the cell C held in the selection container 11 or the microplate 4 from their lower sides, is provided with a lens unit 51 and a camera body 52. The lens unit 51 is an objective lens for use in an optical microscope and includes a lens group which forms an optical image of a predetermined magnification, and a lens barrel which houses the lens group. The camera body 52 is provided with an image pickup element such as a CCD image sensor. The lens unit 51 forms an optical image of an imaging target on a light receiving surface of the image pickup element. The camera unit 5 is movable along a guide rail 5G extending in the right-left direction in parallel with the base 1, and is movable under the base 1 in the X direction and the Y direction. The lens unit 51 is movable in the Z direction for focusing operation.

The head unit 6 is provided for picking up the cell C from the dish 2 and transferring the cell to the microplate 4, and includes the plurality of heads 61, and a head body 62 in which the heads 61 are installed. The tip 12 which conducts suctioning (pick-up) and discharging of the cell C is attached to a distal end of each head 61. The head body 62 holds the head 61 so as to be raised and lowered in +Z and -Z directions and is movable along a guide rail 6G in +X and -X directions. The head body 62 is movable also in the Y direction.

### [Microplate]

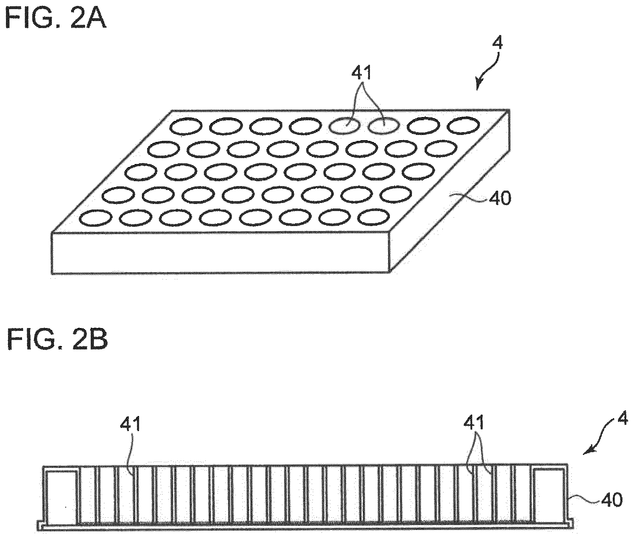

FIG. 2A is a perspective view of the above microplate 4 and FIG. 2B is a vertical sectional view of the microplate 4. The microplate 4 includes a plate body 40 and the plurality of wells 41 aligned in the plate body 40 in a matrix. Since a distal end opening portion t of the tip 12 enters the well 41 during discharge of the cell C, each well 41 has an opening diameter which allows the tip 12 to enter with a margin.

Commercially available microplates have a standard size. A standard microplate has a predetermined length-to-width size (length 85.48 mm × width 126 mm) and has a predetermined number of wells. A general number of wells is 24 × 16 (384 wells), the wells being aligned at a predetermined pitch in a matrix. FIG. 2B shows a sectional view of the microplate 4 with 384 wells. As shown in FIG. 2B, 24 wells 41 are aligned at an equal well pitch in a longitudinal direction of the microplate 4 (16 wells in a shorter side direction).

### [Housing State of Cells in Well]

The cell transfer device S of the present embodiment picks up the cell C satisfying a required condition from the dish 2 and transfers the picked up cell C to the well 41 of the microplate 4. At this time, the cells C are transferred to each of at least two wells 41 among the wells 41 provided in the microplate 4 so that the cells C are housed in a state under the same condition. This is intended to uniformize a housing state of the cell C in each well 41 so as to evaluate a medical effect test and the like in each well 41 under an equivalent condition.

The description "a state under the same condition" can include a mode of, for example, a state where an amount of the cells C housed in each of at least two wells 41 (one example of condition information) is in the same state, or a state where an intensity of fluorescence emitted by the cell C (one example of condition information) is in the same state. The amount of the cells C, which is a physical quantity derived from a shape of the cell C, represents, for example, an area obtained from the cell C two-dimensionally viewed or a volume of the cell C. In a case where a plurality of the cells C are housed in one well 41, the physical quantity represents a total area or a total volume of the cells.

"The same condition" should not be limited to having completely the same amount of the cells C or the same intensity of fluorescence between at least two wells 41. From the beginning, the present embodiment does not consider, as a transfer target, a standardized object such as an industrial product but considers, as a transfer target, a biological subject such as the cell C which inherently varies in an individual size, properties, and the like. Therefore, it is practically impossible to house these targets in at least two wells 41 completely under the same condition. Accordingly, it should be evaluated to be "the same condition" when a variation is within 20%, preferably within 10% in an amount or an intensity of fluorescence of the cells C housed in one well 41 from an amount or an intensity of fluorescence of the cells C housed in the other well 41.

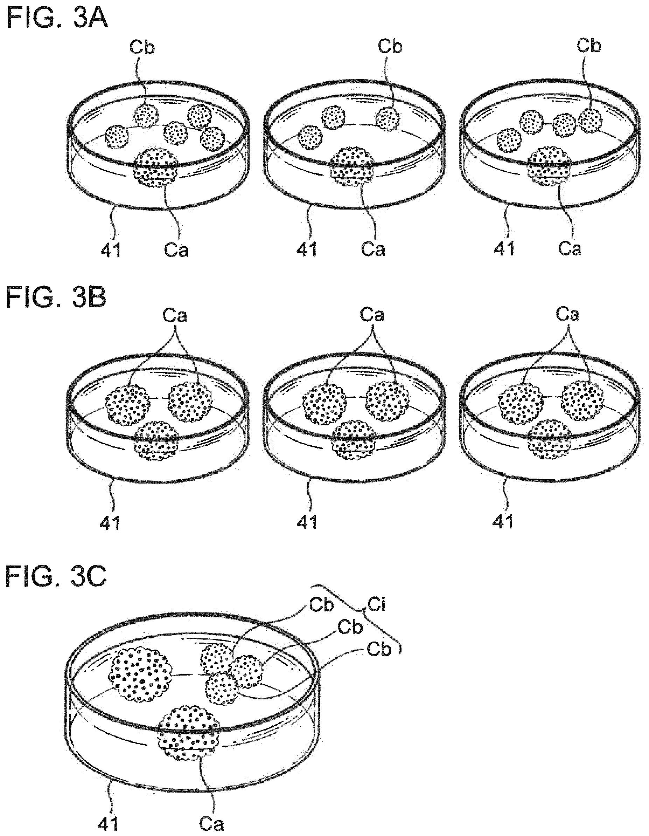

FIG. 3A is a schematic view showing a state of cells housed in the wells 41 in a comparative example. Here, an example is shown in which a cell Ca having a relatively large size and a cell Cb having a relatively small size are housed in three wells 41. In each of the three wells 41, while the cell Ca having a large size is housed one each, the cells Cb having a small size are housed in different numbers. Although it can be said that a housing state of the cells in each well 41 has a similar housing tendency in that one cell Ca having a large size and several cells Cb having a small size are housed, the amounts of the cells cannot be evaluated to have "the same condition" because the number of the small cells Cb varies (three to five).

FIG. 3B is a schematic view showing a state of cells housed in the wells 41 in the present embodiment. Here, an example is shown in which three cells Ca each having a relatively large size are housed in three wells 41. In this example, among the three wells 41, the individual cell has a size of the same grade and the number of cells housed is the same. Accordingly, it is possible to evaluate a housing state of the cells Ca in each well 41 to be a state under the same condition on a precise level.

A housing state of the cells in the well 41 shown in FIG. 3C can be evaluated to be under the same condition as that of the housing state of the cells in each well 41 shown in FIG. 3B. Here, the three small-sized cells Cb collectively form one cell clusters Ci. In a case where the cell clusters Ci is evaluated to be equivalent to one large-sized cell Ca, the well 41 shown in FIG. 3C can be handled as an equivalent of each well 41 in FIG. 3B.

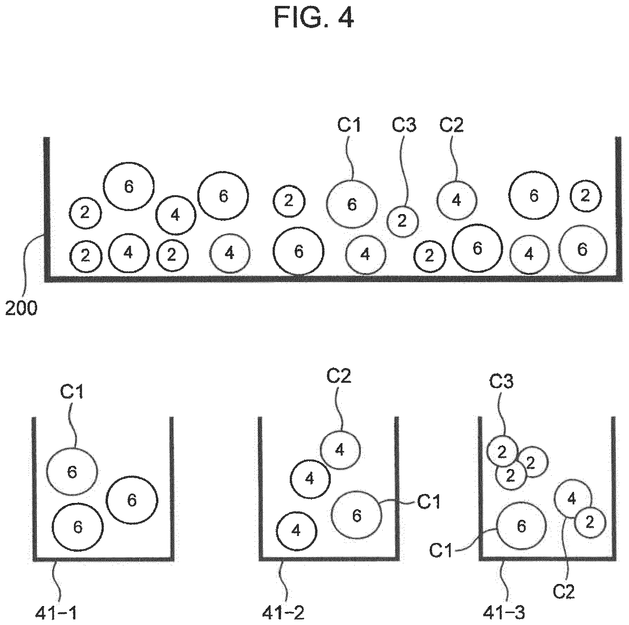

FIG. 4 is a schematic view for describing a concept of bringing an amount of cells C housed in each well 41 into the same state in the present embodiment. As illustrated in FIG. 3B, it is most preferable that the individual cell housed in each well 41 has a size of the same grade and the number of cells housed is the same. However, it may be difficult to realize the state shown in FIG. 3B because three-dimensionally cultured cells tend to have non-uniform shapes. FIG. 4 shows an example of bringing a housing state of cells in the wells 41 into a state under the same state in this case.

In the transfer source container 200 in which a transfer target cell is housed, cells C1, C2, and C3 having different sizes (area or volume), i.e., numerous large-sized cells C1, medium-sized cells C2, and small-sized cells C3 are housed. Numerals "6", "4", and "2" attached to the cells C1, C2, and C3 are set to be evaluation values indicative of sizes of the cells C1, C2, and C3, respectively. Additionally, the cells C1 to C3 are set to be cells selected as cells meeting a condition for a transfer target by an operator. FIG. 4 shows an example in which the cells C1 to C3 are picked up from such transfer source container 200 and cell housing states in three wells 41 - 1, 41-2, and 41-3 are brought into a state under the same condition.

In the first well 41-1, three large-sized cells C1 are housed. The first well 41-1 has an evaluation value of 6 × 3 = 18. In the second well 41-2, one large-sized cell C1 and three medium-sized cells C2 are housed. The second well 41-2 has an evaluation value of (6 × 1) + (4 × 3) = 18. In the third well 41-3, one large-sized cell C1, one medium-sized cell C2, and four small-sized cells C3 are housed. The third well 41-3 has an evaluation value of (6 × 1) + (4 × 1) + (2 × 4) = 18. Thus, although cells housed in the three wells 41-1, 41-2, and 41-3 are different from each other in size and the number, these wells have the same evaluation value for a cell size. In the present embodiment, such a cell housing state as described above is also to be considered as a state under the same condition.

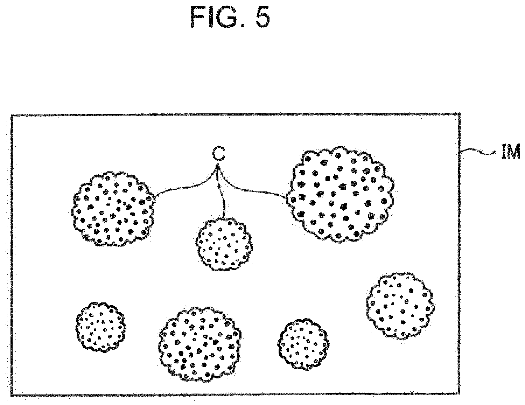

As described above, an amount of the cells C can be evaluated from an area or a volume. These values can be acquired from an image of the cell C captured by the camera unit 5 (the information acquisition unit). FIG. 5 is a view showing an example of a two-dimensional image IM for calculating an area of the cell C. The two-dimensional image IM is acquired by causing the camera unit 5 to capture an image of the dish 2 holding the cell C at, for example, the first mounting position PI (one example of a predetermined holding position) as shown in FIG. 1. By conducting image processing such as pattern recognition processing involving edge detection processing or feature value extraction on the acquired two-dimensional image IM, an external contour shape of the cell C reflected in the two-dimensional image IM can be grasped. An area of each cell C can be calculated based on the shape.

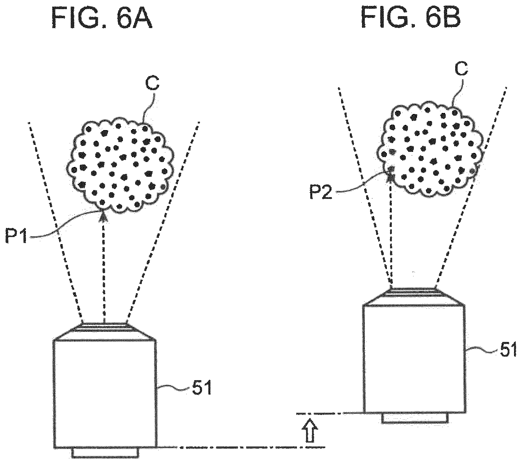

FIGs. 6A and 6B are views for describing a method of capturing an image of the cell C for obtaining a volume of the cell C. The volume of the cell C can be obtained from, for example, an image of the cell C captured by the camera unit 5 at different focal positions. First, as shown in FIG. 6A, first imaging is conducted with a focal position of the lens unit 51 of the camera unit 5 adjusted to the vicinity of a lower portion of the cell C. Next, as shown in FIG. 6B, second imaging is conducted with the focal position of the lens unit 51 adjusted to a portion more to an upper side than the portion in the first imaging. An external contour of each cell C is obtained from an image obtained by these imaging. The same imaging is continued until the external contour becomes the largest. In a case of a spherical cell C, imaging will be conducted n times with respect to a lower hemisphere of the cell. Although imaging of an upper hemisphere of the cell C cannot be conducted, an overall volume of the cell C can be obtained by estimated calculation from an external contour obtained for each of n times of imaging of the lower hemisphere.

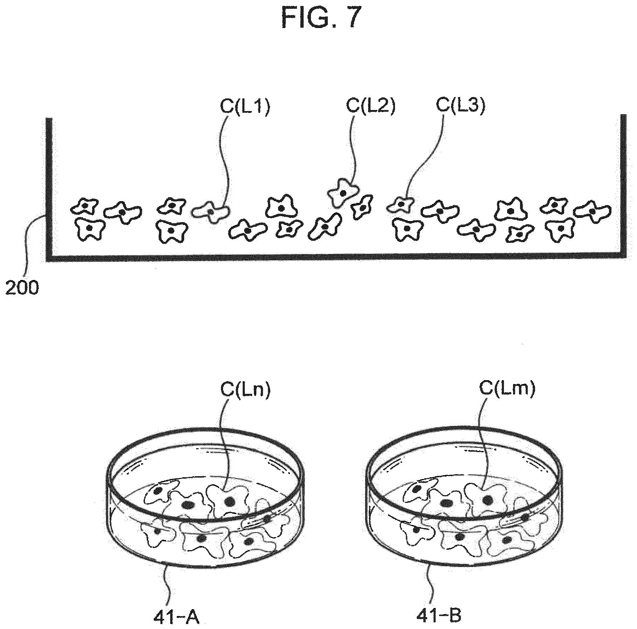

FIG. 7 is a view showing an example of uniformizing the cells C housed in each well 41 based on intensities of fluorescence of the cells C. In a case where an intensity of fluorescence of the cell C is used as condition information, when the cell C has fluorescence, an intensity of the fluorescence is measured. On the other hand, when the cell C does not have fluorescence, by allowing the cell C to react with an appropriate medicine to change the cell C to be fluorescent, an intensity of the fluorescence of the cell C is measured. An intensity of fluorescence can be measured by, for example, a fluorescence analysis device (the information acquisition unit). Alternatively, an intensity of fluorescence can be evaluated by calculating coloring or brightness of each cell C from an image captured by the camera unit 5.

In FIG. 7, numerous cells C (LI), C (L2), and C (L3) ... having different intensities of fluorescence are housed in the transfer source container 200 in which transfer target cells are to be housed. Appended L1, L2, and L3 represent an intensity of fluorescence of each cell C. FIG. 7 shows an example of picking up the cell C from such transfer source container 200 and bringing cell housing states in two wells 41-A and 41-B into a state under the same condition. In the first well 41-A, a plurality of cells C (Ln) each having an inherent intensity of fluorescence Ln are housed. Similarly, in the second well 41-B, a plurality of cells C (Lm) each having an inherent intensity of fluorescence Lm are housed. Then, total intensities of fluorescence of the plurality of cells C(Ln) and total intensities of fluorescence of the plurality of cells C(Lm) are prepared to be the same or approximate (as already described, a difference therebetween is within 20%). For making the total intensities of fluorescence uniform, appropriate cells are selected from the cells C (LI), C(L2), C(L3) ... in the transfer source container 200, and transferred to the first well 41-A and the second well 41-B. This enables a cell housing state in each well 41 to be uniformized even when fluorescence properties vary with individual cells C.

### [Electrical Configuration of Cell Transfer Device]

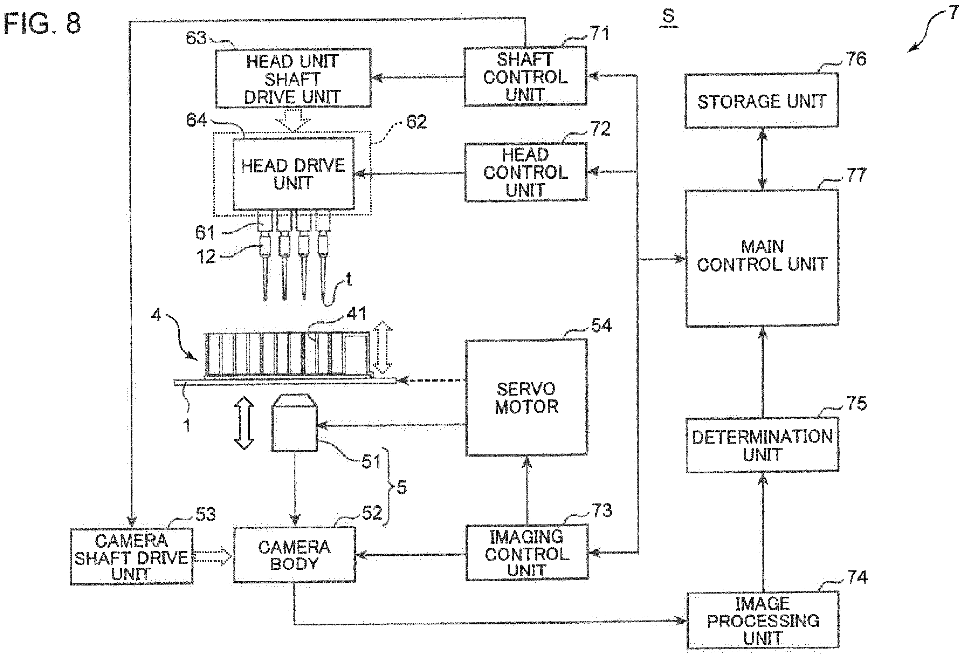

FIG. 8 is a block diagram showing an electrical configuration of the cell transfer device S. The cell transfer device S is provided with a control unit 7 which controls movement of the head unit 6, raising and lowering of the head 61 (the tip 12), cell C suctioning and discharging operations, moving and imaging operations of the camera unit 5, and the like. The cell transfer device S is also provided with a camera shaft drive unit 53 as a mechanism which causes the camera unit 5 to move horizontally, a servo motor 54 as a drive source which causes the lens unit 51 to move up and down, a head unit shaft drive unit 63 as a mechanism which causes the head unit 6 to move horizontally, and a head drive unit 64 as a mechanism which causes the head 61 to be raised and lowered and as a mechanism which causes the head 61 to conduct suctioning and discharging operations.

The camera shaft drive unit 53 includes a drive motor which causes the camera unit 5 to move horizontally along the guide rail 5G (FIG. 1). The camera shaft drive unit 53 causes the camera unit 5 to move between the first mounting position P1 immediately below the dish 2 and the second mounting position P2 immediately below the microplate 4.

Forward rotation or reverse rotation of the servo motor 54 causes the lens unit 51 to move with a predetermined resolution in the up-down direction via a power transmission mechanism (not shown). This movement allows a focal position of the lens unit 51 to be set to the cell C housed in the well 41. As indicated by a dotted line in FIG. 8, not the lens unit 51 but the microplate 4 itself or a stage (the base 1) on which the microplate 4 is mounted can be moved up and down by the servo motor 54. Additionally, although the microplate 4 is illustrated as an imaging target in FIG. 8, the dish 2 becomes an imaging target in a step of acquiring condition information of the cell C (an area of the cell C and the like).

The head unit shaft drive unit 63 includes a drive motor which causes the head unit 6 (the head body 62) to move along the guide rail 6G. The head drive unit 64 includes a motor as a power source which causes the head 61 to be raised and lowered relative to the head body 62, and a mechanism as a power source which causes suction force and discharge force to be generated at the distal end opening portion t of the tip 12.

The control unit 7 is configured with a microcomputer or the like, and functions to include a shaft control unit 71, a head control unit 72, an imaging control unit 73, an image processing unit 74 (a part of the information acquisition unit), a determination unit 75, a storage unit 76, and a main control unit 77 as a result of execution of a predetermined program.

The shaft control unit 71 controls operation of the head unit shaft drive unit 63. Specifically, the shaft control unit 71 causes the head unit 6 to move to a predetermined target position in the horizontal direction by controlling the head unit shaft drive unit 63. Movement of the head 61 (the tip 12) between the selection container 11 and the microplate 4, positioning of the head 61 vertically over the holding recess 3 of the dish 2, and positioning of the head 61 vertically over the well 41 of the microplate 4 as a discharge target, and the like are realized by the control of the head unit shaft drive unit 63 by the shaft control unit 71.

The head control unit 72 causes the head 61 as a control target to be raised and lowered toward a predetermined target position by controlling the head drive unit 64. The head control unit 72 also causes suction force or discharge force to be generated at the distal end opening portion t of the tip 12 at predetermined timing by controlling a suction mechanism corresponding to the head 61 as a control target.

The imaging control unit 73 controls the camera shaft drive unit 53 to control operation of moving the camera unit 5 along the guide rail 5G. The imaging control unit 73 also controls operation (an amount of exposure, shutter timing, etc.) of capturing an image of the dish 2 or the microplate 4 by the camera unit 5. Further, the imaging control unit 73 applies a control pulse to the servo motor 54 to move the lens unit 51 in the up-down direction at a predetermined pitch (e.g. a pitch of several tens µm) for focusing operation.

The image processing unit 74 conducts image processing such as pattern recognition processing involving edge detection processing or feature value extraction on image data acquired by the camera body 52. The image processing unit 74 executes processing of recognizing, on an image, presence (the number) of the cells C on the dish 2 (the holding recess 3), processing of acquiring the X and Y coordinates of each cell C, processing of acquiring condition information of an individual cell C such as external contour, size (area and volume), shape, color tone, etc., and other processing based on an image of the dish 2 after the dispensation of the cell C. Similarly, the image processing unit 74 executes processing of recognizing the number, an amount (a total area or a total volume), an intensity of fluorescence, etc. of the cells C housed in the well 41 based on an image of the well 41 to which the cells C have been transferred.

The determination unit 75 executes various determination processing based on a result of the image processing of the cell C conducted by the image processing unit 74. Specifically, the determination unit 75 selects the cell C which satisfies a condition and is to be transferred to the microplate 4 based on a captured image of the dish 2 holding the cell C. For example, determination is made to exclude, from the transfer target, a cell C having too large or too small a size, a cell C having a shape extremely deformed, and a cell C having color tone considered to be a dead cell or an unhealthy cell.

The determination unit 75 determines whether or not a housing state of the cell C in the well 41 is good based on a captured image of the microplate 4 in which the cell C is housed in the well 41. Particularly, in the present embodiment, the determination unit 75 determines whether or not in at least two wells 41 designated to be in a state under the same condition, each cell C is housed in the state under the same condition.

The storage unit 76 stores various kinds of set values, data, programs, and the like in the cell transfer device S. The storage unit 76 additionally stores feature value data related to a determination criterion of the cell C. The determination unit 75 refers to the feature value data to execute determination of the cell C to be a transfer target.

The main control unit 77 conducts centralized control of operation of the camera unit 5 and the head unit 6. The main control unit 77 controls the camera unit 5 and the head unit 6 through the shaft control unit 71, the head control unit 72, and the imaging control unit 73, so as to transfer the cells C to the microplate 4 by conducting imaging of the dish 2 where the cells C are scattered at the first mounting position PI (FIG. 1) on which the selection container 11 is mounted, and also conducting pick-up of a good cell C selected as a transfer target by the determination unit 75, the pick-up being conducted with suction by the tip 12 attached to the head 61.

At the time of picking up and transferring the cell C, the main control unit 77 refers to condition information (area etc.) of each cell C acquired by the image processing by the image processing unit 74. Then, the main control unit 77 controls pick-up and transfer of cells C so as to be housed in a state under the same condition in at least two wells 41 among the plurality of wells 41 provided in the microplate 4. The state under the same condition is as described above with reference to FIG. 3 to FIG. 7.

The main control unit 77 also controls the camera unit 5 and the head unit 6 so as to conduct, at the second mounting position P2 at which the microplate 4 is mounted, releasing operation of the picked cell C to the well 41 and imaging of the well 41 after the cell C is released. Further, among the wells 41 designated to be in a state under the same condition, the main control unit 77 controls the head unit 6 to add the cell C to or extract the cell C (state operation) from the well 41, determined by the determination unit 75 that the cells C are not housed in the state under the same condition, so as to have the same condition.

### [Operation Flow of Cell Transfer Device]

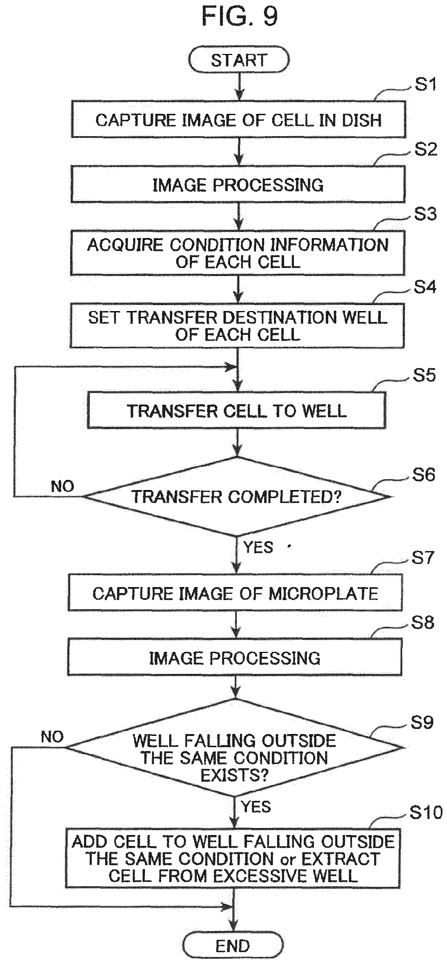

FIG. 9 is a flow chart of cell transfer operation using the cell transfer device S. With reference to FIG. 1 and FIG. 8, when the processing starts, the main control unit 77 causes the camera unit 5 to capture an image of the cell C held in the dish 2 of the selection container 11 at the first mounting position PI (Step S1). Specifically, the main control unit 77 causes the camera shaft drive unit 53 to be driven through the shaft control unit 71 to move the camera unit 5 to a predetermined imaging position at the first mounting position PI. The main control unit also causes the camera body 52 and the servo motor 54 to be driven through the imaging control unit 73 to execute imaging operation while focusing on the cell C on the dish 2.

Subsequently, the image processing unit 74 conducts predetermined image processing with respect to an image acquired by the camera body 52 (Step S2). This image processing specifies a cell C reflected in the acquired image and also derives a feature value (condition information) obtained by converting external contour, size (area and volume), shape, color tone, etc. of an individual cell C into numbers. Then, the determination unit 75 selects a cell C to be transferred to the microplate 4 among the cells C included in the image.

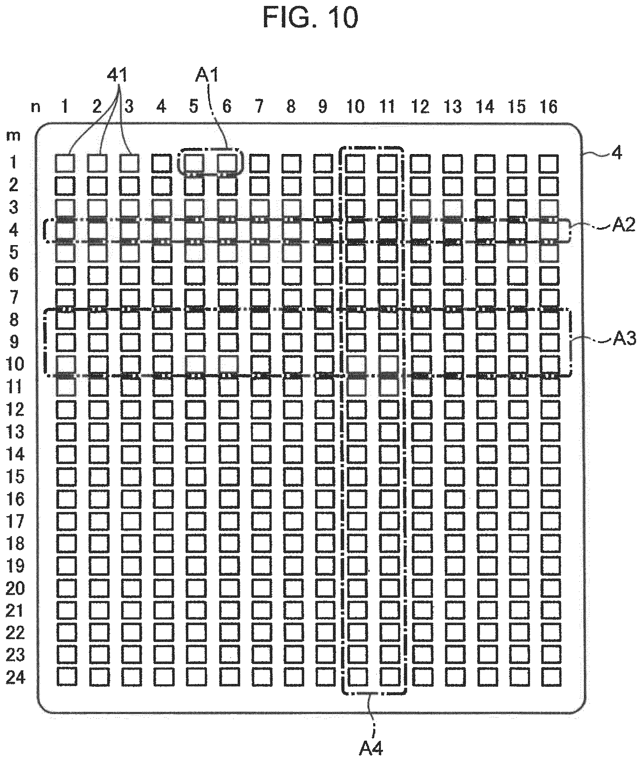

Thereafter, the main control unit 77 acquires condition information of the cell C selected by the determination unit 75 (Step S3). Then, the main control unit 77 sets the well 41 to be a transfer destination of each cell C while referring to the acquired condition information (Step S4). At this time, a transfer destination of each cell C is set so that the cells C are housed in at least two wells 41 in a state under the same condition. FIG. 10 shows a setting example of a cell C transfer destination in the microplate 4.

FIG. 10 is a top view showing one example of the microplate 4, and is a view for describing an example of the wells 41 in which the cells C are housed in a state under the same condition. On the microplate 4, the wells 41 are aligned in m rows by n columns at a predetermined pitch in a matrix. Here, the microplate 4 including m rows × n columns = 24 rows × 16 columns = 384 wells 41 is illustrated. Then, the setting examples A1 to A4 of cell C transfer destinations are shown.

The setting example A1 shows an example in which two adjacent wells 41 (wells 41 at mln5 and mln6) are set as a well group in which the cells C are housed in a state under the same condition. In this case, the same amount (area or volume) of the cells C in total or the same intensity of fluorescence of the cells C in total will be housed in these two wells 41. The setting example A1 is the smallest unit, and three or more adjacent wells 41 can be set as the well group.

The setting example A2 is an example in which among the plurality of wells 41 aligned in a matrix, the wells 41 belonging to one row (m4 row) are set to be a well group of the cells C housed in a state under the same condition. The setting example A3 is an example in which the wells 41 belonging to three adjacent rows (m8 to m10 rows) are set to be a well group of the cells C housed in a state under the same condition. The setting example A4 is an example in which the wells 41 belonging to two adjacent columns (n10 column and n11 column) are set to be a well group of the cells C housed in a state under the same condition. According to such setting examples A1 to A4, since biological subjects are housed in at least two adjacent wells 41, i.e., wells 41 in one or a plurality of rows or columns in a state under the same condition, medicine injection work and the like can be efficiently conducted in a medical effect test and the like. It is also possible to conduct a test for confirming a medical effect of a medicine of, for example, the same kind and the same mixing amount on the above units, or the like.

In addition, all the wells 41 in one microplate 4 can be set as a well group in which the cells C are housed in a state under the same condition. In the microplate 4 illustrated in FIG. 10, all of 384 wells 41 are set to be a well group in which the cells C are housed in a state under the same condition. In this case, various kinds of tests and the like can be conducted with a culture condition (temperature, humidity, etc.) of the cells C changed on a microplate 4 basis.

Returning to FIG. 9, the main control unit 77 sets a transfer sequence of the cell C conducted by the head 61 (the tip 12) according to a transfer destination of each cell C set in Step S4. Specifically, a procedure is set for determining from which of the holding recesses 3 of the dish 2, the cell C is to be sucked by the tip 12 and to which of the wells 41 of the microplate 4, the cell C is to be discharged. Then, the main control unit 77 controls the head unit 6 to transfer the cell C in the dish 2 to the microplate 4 following the transfer sequence (Step S5).

The main control unit 77 checks whether or not transfer of all the cells C selected as transfer targets has been completed (Step S6). In a case where transfer of the cell C is not completed yet (NO in Step S6), cell transfer operation in Step S5 is continued. On the other hand, in a case where transfer of the cell C is completed (YES in Step S6), the main control unit 77 causes the camera unit 5 to capture an image of the microplate 4 after the transfer of the cell C (Step S7). Specifically, the main control unit 77 causes the camera shaft drive unit 53 to be driven through the shaft control unit 71, thereby causing the camera unit 5 to move from the first mounting position PI to the second mounting position P2. The main control unit 77 also causes the camera body 52 and the servo motor 54 to be driven through the imaging control unit 73 to execute imaging operation while focusing on the cell C in the well 41 of the microplate 4.

Subsequently, the image processing unit 74 conducts predetermined image processing with respect to the image acquired by the camera body 52 (Step S8). The determination unit 75 checks a housing state of the cell C in each well 41 with reference to a feature value of the cell C in each well 41 obtained based on the image processing result. Then, the determination unit 75 determines whether or not a well 41 falling outside the same condition exists in a well group set to be a group in which the cell C is housed in a state under the same condition (e.g. well groups in the setting examples A1 to A4 in FIG. 10) (Step S9). In a case where a well 41 falling outside the same condition does not exist (NO in Step S9), the main control unit 77 finishes the processing.

By contrast, in a case where a well 41 falling outside the same condition exists (YES in Step S9), the main control unit 77 causes processing of correcting a cell housing state in such well 41 (operation of a state of a biological subject) to be executed (Step S10). Specifically, in a case where the cause of the cell C in the well 41 falling outside the same condition is a shortage of the cells C, the main control unit 77 controls the head 61 to execute processing of adding an appropriate cell C to the well 41. In a case where the cause of the cell C in the well 41 falling outside the same condition is an excess of the cells C, the main control unit 77 causes execution of processing of extracting an excessive cell C from the well 41. The cell C to be added can be extracted from another well 41 in which excessive cells C are housed. Additionally, the extracted cell C can be added to another well 41 in short of the cells C.

The above operation flow shows an example in which condition information of the cell C is acquired in advance on the dish 2 side as a transfer source so as to allow the cell C to be housed in each well 41 in a state under the same condition. However, there may be a case where an image of the cell C cannot be captured in the container as a transfer source, i.e., a case where condition information of each cell C cannot be acquired in advance. In such a case, at the time of transfer of the cell C from the dish 2 to each well 41 of the microplate 4, a cell housing state in each well 41 can be uniformized on the microplate 4 side after the transfer without consideration of setting an amount of the cells C and the like to be exactly the same. Specifically, by roughly transferring the cell C to each well 41 and thereafter executing Steps S7 to S10 in FIG. 9, the well 41 in a state under the same condition can be formed after the transfer of the cell.

With the transfer method and the cell transfer device S for the cell C according to the present embodiment described in the foregoing, the cells C are housed in at least two wells 41 in a state under the same condition. Accordingly, it is possible to evaluate the cells C in the at least two wells 41 under the same condition by, for example, a medical effect test.

### [Invention Included in Embodiment]

The above-described specific embodiment mainly includes inventions having configurations shown below.

A transfer method for a biological subject according to one aspect of the present invention is a transfer method for a biological subject including picking up a biological subject at a predetermined holding position; and transferring the picked up biological subject to a container having a plurality of wells, in which biological subjects are transferred to at least two wells among the plurality of wells so that the biological subjects are housed in a state under the same condition.

According to this transfer method for a biological subject, since biological subjects are housed in at least two wells in a state under the same condition, the biological subjects can be evaluated in the at least two wells under the same condition.

In the above transfer method for a biological subject, the state under the same condition is desirably a state where amounts of biological subjects housed in the at least two wells are in the same state. In this case, the amount of the biological subjects is desirably an area of the biological subject in a two-dimensional view.

According to this transfer method for a biological subject, an amount (area) of biological subjects can be made uniform in at least two wells. Accordingly, even when individual biological subjects have various shapes, a cell housing state in each well can be uniformized.

In the above transfer method for a biological subject, the state under the same condition is desirably a state where intensities of fluorescence emitted by the biological subjects housed in the at least two wells are in the same state.

According to this transfer method for a biological subject, intensities of fluorescence of biological subjects in at least two wells can be made uniform. Accordingly, even when individual biological subjects have various fluorescence properties, a cell housing state in each well can be uniformized.

A transfer device for a biological subject according to another aspect of the present invention includes an information acquisition unit which acquires condition information of a biological subject at a predetermined holding position; a head having a tip which picks up the biological subject and capable of transferring the picked up biological subject to a container having a plurality of wells; and a control unit which controls operation of the head, in which the control unit refers to condition information of the biological subject acquired by the information acquisition unit and causes the head to transfer biological subjects to at least two wells among the plurality of wells so that the biological subjects are housed in a state under the same condition.

According to this transfer device for a biological subject, the control unit can acquire condition information of a biological subject from the information acquisition unit before transfer of the biological subject to a well. By controlling operation of the head with reference to this condition information, the control unit enables biological subjects to be housed in at least two wells in a state under the same condition. Accordingly, it is possible to evaluate biological subjects in the at least two wells under the same condition.

In the above transfer device for a biological subject, the information acquisition unit desirably acquires an amount of the biological subjects as the condition information. This enables amounts of biological subjects in at least two wells to be made uniform. Accordingly, even when individual biological subjects have various shapes, a cell housing state in each well can be uniformized.

In the above case of acquiring an amount of biological subjects, the information acquisition unit is an imaging unit which captures an image of a biological subject at the holding position, and the amount of the biological subjects may be an area of the biological subjects in a captured image.

Alternatively, the information acquisition unit is an imaging unit which captures an image of a biological subject at the holding position, and the amount of the biological subjects may be a volume of the biological subjects obtained from an image of the biological subject captured by the imaging unit at different focal positions.

Additionally, the information acquisition unit may be configured to acquire an intensity of fluorescence emitted by a biological subject as the condition information.

In the above transfer device for a biological subject, the control unit desirably causes the head to transfer biological subjects so as to be housed in at least two adjacent wells in a state under the same condition among the plurality of wells provided in the container.

According to this transfer device for a biological subject, since biological subjects are housed in at least two adjacent wells in a state under the same condition, medicine injection work and the like can be efficiently conducted in a medical effect test and the like.

In the above transfer device for a biological subject, desirably, the container includes a plurality of wells aligned in a matrix, and the control unit causes the head to transfer biological subjects so as to be housed in a well belonging to at least one row or one column in a state under the same condition among the plurality of wells aligned in a matrix.

According to this transfer device for a biological subject, it is also possible to conduct a test for confirming a medical effect of a medicine of, for example, the same kind and the same mixing amount in units of a well in one row or in one column.

In the above transfer device for a biological subject, the control unit may also cause the head to transfer biological subjects so as to be housed in all the wells of the plurality of wells provided in the container in a state under the same condition.

The above transfer device for a biological subject desirably further includes an imaging unit which captures an image of the container in a state of having the biological subjects housed in the wells.

According to this transfer device for a biological subject, the imaging unit enables monitoring of a result of work for transferring a biological subject to the container. In other words, it is possible to evaluate whether or not a biological subject is housed in each well in a state under the same condition.

In this case, it is desirable that the transfer device for a biological subject further includes a determination unit which determines whether or not the biological subjects are housed in the wells in a state under the same condition based on an image captured by the imaging unit, in which the control unit controls the head to execute state operation of the biological subject for a well determined as not being in the state under the same condition by the determination unit.

According to this transfer device for a biological subject, the state operation of the biological subject is conducted for a defective well determined as not being in the state under the same condition. Therefore, it is possible to correct a housing state of a biological subject in a defective well.

According to the present invention described in the foregoing, it is possible to provide a transfer method and a transfer device for a biological subject, which enable evaluation of a biological subject in each well of a plurality of wells under the same condition when biological subjects are transferred to the plurality of wells to conduct processing such as a medical effect test.

## Claims

1. A transfer method for a biological subject comprising:
picking up a biological subject at a predetermined holding position; and
transferring the picked up biological subject to a container having a plurality of wells,
wherein biological subjects are transferred to at least two wells among the plurality of wells so that the biological subjects are housed in a state under the same condition.

2. The transfer method for a biological subject according to claim 1, wherein the state under the same condition is a state where amounts of biological subjects housed in the at least two wells are in the same state.

3. The transfer method for a biological subject according to claim 2, wherein the amount of the biological subjects is an area of the biological subject in a two-dimensional view.

4. The transfer method for a biological subject according to claim 1, wherein the state under the same condition is a state where intensities of fluorescence emitted by the biological subjects housed in the at least two wells are in the same state.

5. A transfer device for a biological subject comprising:
an information acquisition unit which acquires condition information of a biological subject at a predetermined holding position;
a head having a tip which picks up the biological subject and capable of transferring the picked up biological subject to a container having a plurality of wells; and
a control unit which controls operation of the head,
wherein the control unit refers to condition information of the biological subject acquired by the information acquisition unit and causes the head to transfer biological subjects to at least two wells among the plurality of wells so that the biological subjects are housed in a state under the same condition.

6. The transfer device for a biological subject according to claim 5, wherein the information acquisition unit acquires an amount of the biological subjects as the condition information.

7. The transfer device for a biological subject according to claim 6, wherein
the information acquisition unit is an imaging unit which captures an image of a biological subject at the holding position, and
the amount of the biological subjects is an area of the biological subjects in a captured image.

8. The transfer device for a biological subject according to claim 6, wherein
the information acquisition unit is an imaging unit which captures an image of a biological subject at the holding position, and
the amount of the biological subjects is a volume of the biological subjects obtained from an image of the biological subject captured by the imaging unit at different focal positions.

9. The transfer device for a biological subject according to claim 5, wherein the information acquisition unit is configured to acquire an intensity of fluorescence emitted by a biological subject as the condition information.

10. The transfer device for a biological subject according to any one of claims 5 to 9, wherein the control unit causes the head to transfer biological subjects so as to be housed in at least two adjacent wells in a state under the same condition among the plurality of wells provided in the container.

11. The transfer device for a biological subject according to any one of claims 5 to 9, wherein
the container includes a plurality of wells aligned in a matrix, and
the control unit causes the head to transfer biological subjects so as to be housed in a well belonging to at least one row or one column in a state under the same condition among the plurality of wells aligned in a matrix.

12. The transfer device for a biological subject according to any one of claims 5 to 9, wherein the control unit causes the head to transfer biological subjects so as to be housed in all the wells of the plurality of wells provided in the container in a state under the same condition.

13. The transfer device for a biological subject according to any one of claims 5 to 12, further comprising an imaging unit which captures an image of the container in a state of having the biological subjects housed in the wells.

14. The transfer device for a biological subject according to claim 13, further comprising:
a determination unit which determines whether or not the biological subjects are housed in the wells in a state under the same condition based on an image captured by the imaging unit,
wherein the control unit controls the head to execute state operation of the biological subject for a well determined as not being in the state under the same condition by the determination unit.
